# EUROPEAN PATENT APPLICATION

(11) **EP 0 853 931 A2**
(43) Date of publication of application: **22.07.1998**
(21) Application number: 97122707.9
(22) Date of filing: 22.12.1997
(51) Int. Cl.: A61F 2/36, B21D 41/02, A61F 2/46

(54) **Distal terminal element for the stem of a primary hip prosthesis**

(30) Priority: 17.01.1997 IT BO970021
(71) Applicant: Orsi, Stefano, Jardin Vitoria Regia 05657080, Sao Paulo-SP (BR)
(72) Inventor: Orsi, Stefano, Jardin Vitoria Regia 05657080, Sao Paulo-SP (BR)
(74) Representative: Modiano, Guido, Dr.-Ing.

(57) **Abstract**

A distal terminal element (1) for the stem of a primary hip prosthesis (P), which over a short extent is tubular (2) and is affected by at least two radial longitudinal slots (3), which form at least two sectors (4) suitable to be flared outward symmetrically before installation in order to assume preset external dimensions which are greater than those of the stem (16) and substantially correspond to the dimensions of the affected medullary canal (C).

## Description

The present invention relates to a distal terminal element for the stem of a primary hip prosthesis of the non-cemented type.

Conventionally, the stems of hip prostheses must be rigidly coupled in the medullary canal of the femur after removing the neck at the upper proximal region and instead they simply need to be stabilized at the lower distal end. In practice, stresses are discharged mostly at the rigidly coupled proximal region, while in the distal region it is simply necessary to avoid small relative movements between the apex of the prosthesis and the bone.

In order to avoid such relative movements in the distal region, sometimes the dimensions of the femoral canal are adapted, by successive reaming operations, to the dimensions of the stem of the prosthesis: this requires a nonbiological and prolonged intervention, increases bleeding and possibly facilitates infections and heterotrophic ossifications.

Another method consists in providing a plurality of prostheses with stems provided with a distal part having different diameters (for example five or six or more diameters), but this entails (since the proximal characteristics of the prosthesis also require the availability of a set of four or five or more different types) the need to have a very large range of sizes, which entails high manufacturing and storage costs.

Modular prostheses are also known which consist of modular components which are mutually assembled before installation; however, this solution entails high costs, some difficulty in assembly and the risk of metallosis due to the various metal to metal couplings.

Components are also known in which an external adapter is applied to the distal end of the stem: said adapter is roughly olive-shaped and axially perforated and can be selected before installation among a plurality of olives having different outside diameters. This solution, however, entails the drawback that the olive, in the course of time, can detach and slip off the stem if the stem has to be removed.

The aim of the present invention is to obviate the above-cited drawbacks of the prior art, i.e., to provide a distal terminal element for the stem of a primary hip prosthesis which does not require particular intensive interventions, can assume different dimensions according to requirements without resorting to a large range of stems, and does not entail the presence of detachable components.

Within the scope of this aim, an object of the present invention is to provide a structure which is simple, relatively easy to provide in practice, safe in use, effective in operation and has a relatively low cost.

This aim, this object and others which will become apparent hereinafter are achieved by a distal terminal element for a stem of a primary hip prosthesis, characterized in that over a short extent it is tubular and is provided with at least two radial longitudinal slots, which form at least two sectors suitable to be flared outward symmetrically before installation in a medullary canal in order to assume preset external dimensions which are greater than those of the stem and substantially correspond to the dimensions of the affected medullary canal.

Further characteristics and advantages of the present invention will become apparent from the following detailed description of a preferred but not exclusive embodiment of a distal terminal element for the stem of a primary hip prosthesis according to the invention, illustrated only by way of non-limitative example in the accompanying drawings, wherein:
figures 1A and 1B are two perspective views of a distal terminal element for the stem of a primary hip prosthesis, in the condition before and after flaring, respectively;
figure 2 is a schematic side view of a distal terminal element according to the present invention and of a corresponding flaring element;
figure 3 is a schematic side view of a distal terminal element according to the present invention and of a corresponding flaring element which is an alternative to the one shown in figure 2;
figure 4 is a schematic perspective view of a distal terminal element according to the present invention and of a corresponding flaring element which is an alternative to those shown in figures 2 and 3;
figure 5 is a schematic perspective view of a distal terminal element according to the present invention and of a corresponding flaring element which is an alternative to those of figures 2, 3 and 4;
figure 6 is a schematic perspective view of a distal terminal element according to the present invention and of a corresponding flaring element which is an alternative to those shown in figures 2, 3, 4 and 5;
figure 7 is a schematic side view of a prosthesis implanted in a femur;
figure 8 is a schematic perspective view of the set of instruments for measurement and adaptation to the femoral canal before application.

With particular reference to the above figures, the reference numeral 1 generally designates a distal terminal element for the stem of a primary hip prosthesis, generally designated by the reference letter P, according to the invention.

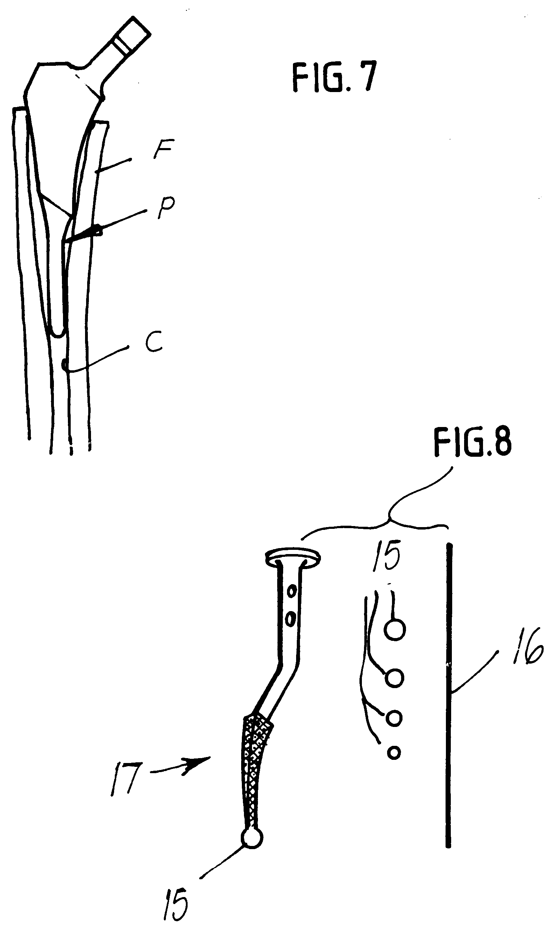

The letter F designates the upper part of the femoral bone, from which the neck has been removed, and the letter C designates the medullary canal, as shown in figure 7.

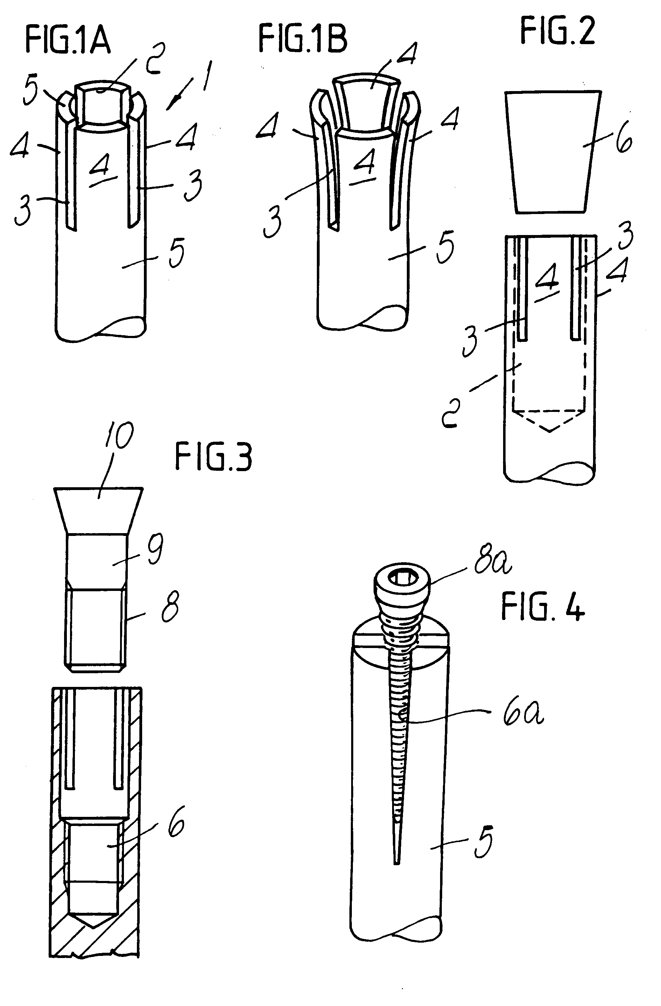

The present invention relates to the lower distal end of the prosthesis P which, over a short extent (for example 20 millimeters), is tubular, 2, and is affected by at least two radial longitudinal slots 3 (in figures 1A and 1B illustrated by way of example there are four slots, but the number might also be different) which form at least two sectors 4 (four in the illustrated case).

The sectors 4 are meant to be flared out and widened symmetrically outward, see figure 1B, before the installation of the prosthesis in the femoral bone in order to assume preset outside dimensions which are greater than those of the stem and are substantially equal to those of the medullary canal, which is measured beforehand and optionally treated: advantageously, the edge 5 of the distal end is slightly rounded so as to prevent sharp corners from making contact with the internal cortex of the medullary canal.

The sectors 4 can be flared outward in any manner. Some solutions for performing outward flaring, given merely by way of example, are shown schematically in figures 2, 3, 4, 5 and 6; the ends of the slots are affected by larger holes 7 which are suitable to facilitate the flaring of the sectors 4.

In the example of figure 2, the sectors 4 are plastically deformed by inserting and axially forcing a frustum-shaped tool 6 which can be removed after deformation: the tool 6 is associated with a sturdy lever arm on which one acts manually before installation, after clamping the stem in a pair of supporting jaws: in another embodiment, the tool 6 is associated with the threaded stem of a screw press.

In the embodiment shown in figure 3, the tubular portion 2 extends beyond the slots 3 with a threaded portion 6 in which an end screw 8 of a stem 9 is coupled; the stem ends with a diverging frustum-shaped portion 10; the screw coupling of portions of longer or shorter lengths of the stem produces a more or less extensive flaring of the sectors 4.

According to requirements, the screw can be removed from the stem before insertion in the femoral canal or can remain rigidly coupled to the stem, optionally by deforming the thread of the screw.

In another embodiment, shown in figure 4, the threaded hole 6a in the stem is frustum-shaped and the screw 8a has a conical stem so that it directly opens out the sectors 4 with its body during penetration.

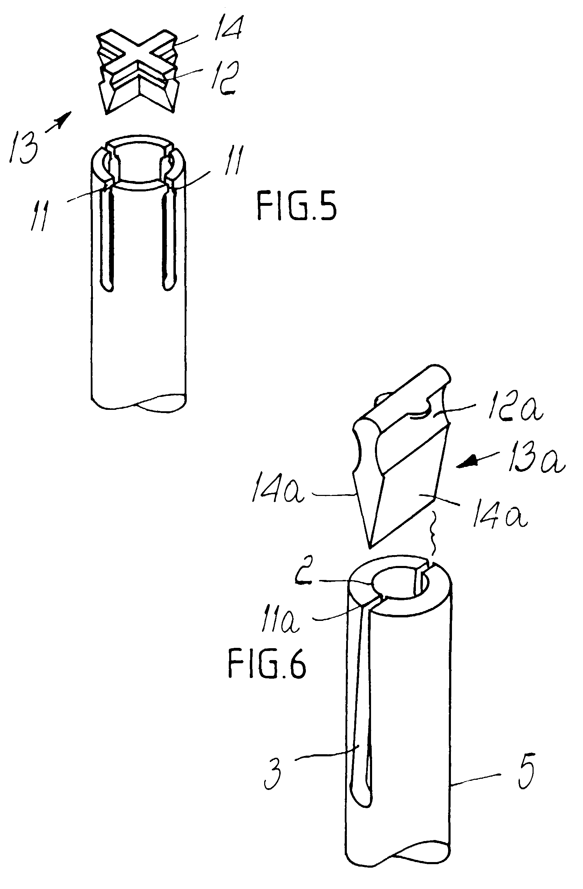

In another embodiment, shown in figure 5, the radial longitudinal slots 3 are thinner proximate to the end, so as to form mutually opposite pairs of coupling teeth 11 which are meant to fit, by snap action, in corresponding notches 12 of a terminal element 13 which is shaped like a star, provided with four arms in the particular case, with wedges 14 which are suitable to be forced axially into the slots 3 of the tubular end of the terminal element in order to flare out the sectors 4. Depending on the larger or smaller dimensions of the outside diameter that the terminal element is to have, star-shaped sets of wedges with more or less deep notches are inserted.

In an alternative embodiment, shown in figure 6, the radial longitudinal slots 3 are shaped like an elongated drop. A terminal element 13a which is shaped like a wedge is provided with surfaces 14a which join at the vertex of the wedge and are suitable to be forced axially into the slots 3 of the tubular end of the terminal element in order to flare out the sectors 4. Depending on the larger or smaller dimensions of the outside diameter that the terminal element must have, the wedge 13a has a different thickness. Protruding portions 12a are provided in the upper region of the wedge to engage the tubular element 2 when the wedge is inserted in the slots 3.

Figure 7 illustrates the different components of the set of instruments that has been devised for measurement and adaptation to the femoral canal before application: the set of instruments comprises a plurality of specimen balls 15 with increasing diameters, which can be rigidly coupled by screwing to the threaded end of a stem 16, provided with graduation notches, which allows to measure the cross-section of the femoral canal and the relative distance from the upper end of the bone: once the ball that has the same diameter and dimensions as the canal has been located, the ball is fixed to the end of a rasp 17 (chosen among a plurality of increasingly larger rasps) which is shaped so as to allow to act on the proximal end of the bone with the rasp without interfering with the bone in a distal position during the preparation of the seat for the proximal part of the prosthesis; in some cases it may be necessary to widen the femoral canal in the distal region: thereafter, the appropriate ball is replaced at the end of the rasp.

After measuring the diameter of the medullary canal and after preparing the proximal region of the bone according to its anatomy and therefore according to the chosen prosthesis, on the basis of the most suitable proximal size and shape characteristics, the sectors 4 of the stem are flared outward with any method, for example one of those shown in figures 2, 3, 4, 5 and 6, until the outside diameter of the distal end is equal to the diameter of the determined specimen ball: the value of the outside diameter reached by means of the deformation is then checked with a go-not-go system, for example, which is constituted by tubular specimen pipes with increasing diameters: once the prosthesis has been implanted, the distal end is therefore highly stabilized in the medullary canal.

It has thus been shown that the present invention achieves the intended aim and objects and in particular that a distal terminal element for the stem of a primary hip prosthesis has been provided which does not require particularly intensive interventions, can assume different dimensions according to requirements without requiring a wide range of stems (in practice, a single stem for each kind of prosthesis with different proximal characteristics) and does not entail the presence of detachable components.

The invention thus conceived is susceptible of numerous modifications and variations, all of which are within the scope of the inventive concept.

All the details may also be replaced with other technically equivalent ones.

In practice, the materials employed, as well as the shapes and the dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A distal terminal element for a stem of a primary hip prosthesis, characterized in that over a short extent it is tubular and is provided with at least two radial longitudinal slots, which form at least two sectors suitable to be flared outward symmetrically before installation in a medullary canal in order to assume preset external dimensions which are greater than those of the stem and substantially correspond to the dimensions of the affected medullary canal.

2. A terminal element according to claim 1, characterized in that said sectors are deformed plastically by means of the axial insertion and forcing of a removable frustum-shaped tool.

3. A terminal element according to claim 1, characterized in that said tubular portion is internally threaded for coupling to a screw whose screwing produces the flaring of said sectors.

4. A terminal element according to claim 3, characterized in that a stem is rigidly coupled to the screw and ends with a diverging frustum-shaped portion.

5. A terminal element according to claim 4, characterized in that said tubular portion has a frustum-shaped threaded hole and said screw has a conical shank.

6. A terminal element according to claim 3, characterized in that said screw is removed from the stem before insertion in the femoral canal.

7. A terminal element according to claim 1, characterized in that said radial longitudinal slots, proximate to the distal end, become narrower so as to form teeth for coupling at corresponding notches of a terminal element which is shaped like a star with wedges which are suitable to be forced axially into said tubular end in order to flare out said sectors.

8. A terminal element according to claim 1, characterized in that the ends of said slots are affected by holes which have an enlarged diameter and are suitable to facilitate the flaring out of said sectors.

9. A set of instruments for measurement and adaptation to a femoral canal before application of a terminal element according to claim 1, comprising a plurality of balls of increasing diameter which can be rigidly coupled to the end of a graduated stem for measuring the femoral canal and to the end of rasps which are shaped so as to not interfere with the bone in the distal position during the preparation of the seat in a proximal position.
